# EUROPEAN PATENT APPLICATION

(11) **EP 4 512 898 A1**
(43) Date of publication of application: **26.02.2025**
(21) Application number: 22963879.6
(22) Date of filing: 02.11.2022
(51) Int. Cl.: C12N 15/113, C12Q 1/6806, C40B 50/06

(54) **BLOCKING SEQUENCE, KIT THEREOF, AND METHOD FOR USING SAME**

(71) Applicant: Boe Technology Group Co., Ltd., Beijing 100015 (CN)
(72) Inventor: YE, Bangquan, Beijing 100176 (CN)
(74) Representative: Durán-Corretjer, S.L.P.
(86) International application number: PCT/CN2022/129312
(87) International publication number: WO 2024/092562

(57) **Abstract**

Provided is a blocking sequence, a kit thereof, and a method for using same. The blocking sequence comprises a blocking sequence 1, a blocking sequence 2, a blocking sequence 3, and a blocking sequence 4. The blocking sequence 1 is complementary to a sequencing joint P5 and used for blocking the sequencing joint P5 at the 5' terminal of a first chain when a capture library is prepared in a target area. The blocking sequence 2 is complementary to an Rspl sequence and used for blocking the Rspl sequence in a 5'-to-3' direction on the first chain when the capture library is prepared in the target area. The blocking sequence 3 is partially complementary to an Rsp2' sequence and used for blocking the Rsp2' sequence in a 5'-to-3' direction on a second chain when the capture library is prepared in the target area. The blocking sequence 4 is complementary to a sequencing joint P7' and used for blocking the sequencing joint P7' at the 5' terminal of the second chain when the capture library is prepared in the target area. The blocking sequence can promote blocking and effective complementary pairing with joint sequences, improve hybridization specificity, and significantly reduce the cross-contamination rate.

## Description

### Sequence Listing

The present application includes a Sequence Listing which has been submitted electronically in ST.26 XML format and hereby incorporated by reference in its entirety. The ST.26 XML copy above, created on October 27, 2022, is named 182211602_sequence_listing_20221027. XML, and is 40kb in size.

### Technical Field

The present disclosure relates to the technical field of gene sequencing library preparation, and specifically to blockers of a target region capture library, kits and methods of use thereof.

### Background

Target region hybridization capture technology refers to the process of customizing target region specific probes for one or more gene sequences, subjecting the specific probes to hybridizing with genomic DNA and enriching target gene DNA fragments. Specifically, it includes genomic DNA fragmentation, terminal repair and A base addition, connecting to adapter sequences and carrying out the first round of PCR amplification to obtain the whole genome library pre-library (whole genome library), then hybridizing the pre-library with the target region specific probe, cleaning the hybridized products and carrying out the second round of PCR amplification to obtain the final capture library.

### Summary

The following is a summary of subject matter described herein in detail. The summary is not intended to limit the protection scope of claims.

Embodiments of the present disclosure provide blockers, kits and methods of use thereof.

The embodiment of the present disclosure provides a blocker, comprising blockers 1, 2, 3 and 4; wherein
the blocker 1 is complementary to a sequence of sequencing adapter P5 for blocking the sequence of sequencing adapter P5 at the 5' end of a first strand during the preparation of a target region capture library;
the blocker 2 is complementary to a sequence of Rspl for blocking the sequence of Rspl in 5' end to 3' end direction on the first strand during the preparation of the target region capture library;
the blocker 3 is partially complementary to a sequence of Rsp2' for blocking the sequence of Rsp2' in 5' end to 3' end direction on a second strand during the preparation of the target region capture library;
the blocker 4 is complementary to a sequence of sequencing adapter P7' for blocking the sequence of sequencing adapter P7' at the 5' end of the second strand during the preparation of the target region capture library.

In some exemplary embodiments, the 3' ends of the blockers 1 and 2 correspond to the 5' end of the first strand, and when the blockers 1 and 2 are used as primers, no insert is introduced and contamination can be reduced; and the blockers 3 and 4 correspond to the 5' end of the second strand, and when the blockers 3 and 4 are used as primers, no insert is introduced and contamination can be reduced.

In some exemplary embodiments, the blocker 1 comprises a locked nucleic acid at position 25 base from the 5' end; the blockers 2 and 3 comprise an reverse dT modification at the 3' end; and the blocker 4 comprises locked nucleic acids at positions 20 and 22 bases from the 5' end.

In some exemplary embodiments, the blocker 3 is complementary to the Rsp2' sequence from the first base, at the 5' end, to the 27th base, at the 3' end direction.

In some exemplary embodiments, the blocker 1 is 28 bases in length, the blocker 2 is 33 bases in length, the blocker 3 is 27 bases in length, and the blocker 4 is 24 bases in length.

In some exemplary embodiments, the blocker 1 comprises the sequence set forth in SEQ ID No. 1, the blocker 2 comprises the sequence set forth in SEQ ID No. 2, the blocker 3 comprises the sequence set forth in SEQ ID No. 3 or SEQ ID No. 5, and the blocker 4 comprises the sequence set forth in SEQ ID No. 4.

Embodiments of the present disclosure also provide a capture kit, comprising the blockers above.

Embodiments of the present disclosure also provide a method for capturing a target region library, comprising capturing a target region from a library to be captured using the blockers or the capture kit above.

In some exemplary embodiments, the sample is fragmented prior to capturing the target region from the library to be captured; and, the fragments of the sample have an average length ranging from 200bp to 250bp.

In some exemplary embodiments, the method further includes subjecting the fragments of the sample to pre-library preparation.

In some exemplary embodiments, the method further includes adding an index sequence to the fragments of the sample.

The embodiment of the disclosure also provides a method for constructing a target region capture library, comprising the following steps:
fragmenting sequences of a sample;
preparing a pre-library;
preparing the target region capture library from the pre-library using the blockers or the capture kit above to obtain the target region capture library;
performing PCR on the target region capture library to obtain a sequencing library.

In some exemplary embodiments, the method includes:
fragmenting the sample into fragments of the sample with an average length ranging from 200bp-250bp;
adding an index sequence to the fragments of the sample;
blocking the fragments of the sample using the blockers or the capture kit;
performing PCR on the sequences blocked to obtain the sequencing library.

In some exemplary embodiments, when preparing a target region capture library, the molar ratio of the blockers to the sequences in the pre-library is (150: 1)-(160: 1), preferably (152: 1)-(157: 1), preferably (153: 1)-(156: 1), preferably 154, preferably 155.

Other aspects may be understood upon reading and understanding the drawings and detailed description.

### Brief Description of Drawings

Accompanying drawings are used for providing further understanding of technical solutions of the present disclosure, constitute a part of the specification, and are used for explaining the technical solutions of the present disclosure together with the embodiments of the present disclosure, but do not constitute limitations on the technical solutions of the present disclosure. Shapes and sizes of one or more components in the drawings do not reflect true scales, and are only intended to schematically describe the contents of the present disclosure.
Fig. 1 shows structures of the adapter sequences for a single-stranded library.
Fig. 2 shows nonspecific binding of the adapter sequences of a single-stranded library.
Fig. 3 shows a structure figure of an exemplary technical scheme of the present disclosure.
Fig. 4 shows a structure figure of blocking of P5 sequence at 5' end of the first strand by blocker 1 of an exemplary technical scheme of the present disclosure.
Fig. 5 shows a structure figure of blocking of Rspl sequence in 5' end to 3' end direction of the first strand by blocker 2 of an exemplary technical scheme of the present disclosure.
Fig. 6 shows a structure figure of partially blocking of Rsp2' sequence in 5' end to 3' end direction of the second strand by blocker 3 of an exemplary technical scheme of the present disclosure.
Fig. 7 shows a structure figure of blocking of P7 sequence at 5' end of the second strand by blocker 4 of an exemplary technical scheme of the present disclosure.
Fig. 8 shows a structure figure of the locked nucleic acid of an exemplary technical scheme of the present disclosure.
Fig. 9 shows a structural figure of a scheme of blocker with an N base.
Fig. 10 shows technical scheme of the blocking positions both being at the 3' end of two strands of DNA in library.
Fig. 11 shows the presence of contamination in blockers containing an index.
Fig. 12 shows a structure figure of a scheme of blocker with C3 spacer modification.
Fig. 13 shows a structure figure of completely blocking of Rsp2 sequence in 5' end to 3' end direction of the second strand by blocker 3 of an exemplary technical scheme of the present disclosure.
Fig. 14 shows a comparison of the results of the library preparation of an exemplary technical scheme of the present disclosure to non-blockers, N-base blockers, and C3 spacer blockers.
Fig. 15 shows a comparison of capture efficiency in the library preparation of an exemplary technical scheme of the present disclosure to non-blockers, N-base blockers, and C3 spacer blockers.
Fig. 16 shows a comparison of EGFR mutation frequencies of the library preparation of an exemplary technical scheme of the present disclosure to non-blockers, N-base blockers, and C3 spacer blockers.

### Detailed Description

To make the objectives, technical solutions, and advantages of the present disclosure clearer, the embodiments of the present disclosure will be described in detail below. It is to be noted that the embodiments in the present disclosure and features in the embodiments may be randomly combined with each other if there is no conflict.

The embodiments of the present disclosure will be described below with reference to the drawings in detail. Implementations may be implemented in a plurality of different forms. Those of ordinary skills in the art may easily understand such a fact that implementations and contents may be transformed into one or more forms without departing from the purpose and scope of the present disclosure. Therefore, the present disclosure should not be explained as being limited to contents described in following implementations only. The embodiments in the present disclosure and features in the embodiments may be combined randomly with each other if there is no conflict.

All technical and scientific terms used herein have the same meanings as understood by those of ordinary skill in the art to which the present disclosure pertains, unless otherwise defined. Although any methods and materials similar or equivalent to those described herein may be used to practice or test the present disclosure, preferred methods and materials are described. For the purposes of the present disclosure the following terms are defined hereinafter.

In this application, unless otherwise explicitly stated, the use of the singular includes the plural. It must be noted that the singular forms "a", "an" and "the" as used in this specification include plural referents, unless the context clearly dictated otherwise. In this application, unless otherwise stated, the use of "or" means "and/or".

The terms "about" or "approximately" mean to be within an acceptable error range of a particular value, as determined by one of ordinary skill in the art, which will depend in part on how the value is measured or determined, i.e. the limitations of the measurement system. For example, "about" may mean to be within one or more than one standard deviation according to the practice in the art. Alternatively, "about" may mean a range of up to 20*%*, up to 10*%*, up to 5*%*, or up to 1*%* of a particular value. In other examples, an amount of "about 10" includes 10 and any amount from 9 to 11.

In yet other instances, the term "about" referring to a reference value may also include a range of values of plus or minus 10*%*, 9*%*, 8*%*, 7*%*, 6*%*, 5*%*, 4*%*, 3*%*, 2*%*, or 1*%* of that value. Alternatively, particularly in relation to biological systems or processes, the term "about" may mean to be within an order of magnitude of a value. When specific values are described in the present application and claims, the term "about" is assumed to mean to be within an acceptable margin of error for the specific values unless otherwise stated.

As use in this specification and one or more claims, the words "comprising" (and any form of comprising, such as "comprise" and "comprises"), "having" (and any form of having , such as "have" and "has"), "including" (and any form of including, such as "include" and "includes") or "containing" (and any form of containing, such as "contains" and "contain") are inclusive or open, and do not exclude additional, unlisted elements or method steps. It is contemplated that any of the embodiments discussed in this specification may be implemented with reference to any method or combination of the present disclosure and vice versa. In addition, the combination of the present disclosure may be used to implement the method of the present disclosure.
Index: Sample label, which refers to the ID of each sample
Blocker: blocking sequence
Rsp: Read Sequencing Primer: Sequencing Primer

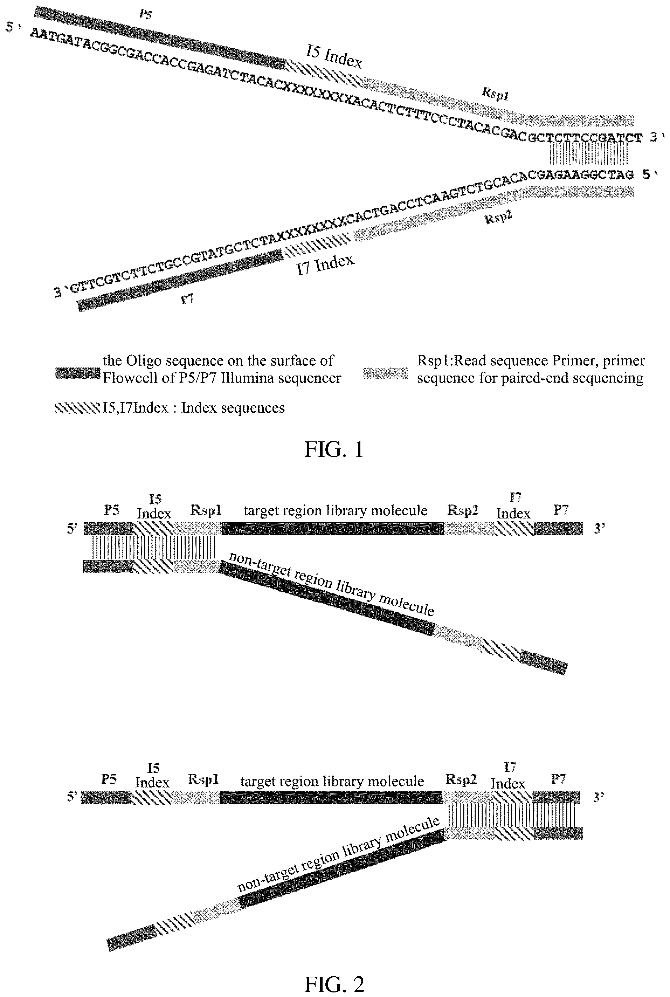

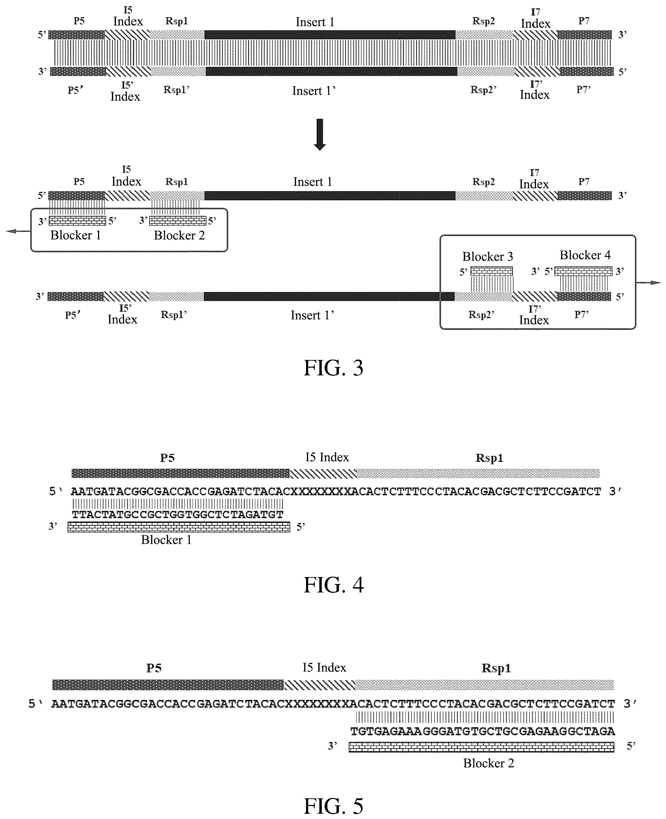

Target region hybridization capture technology refers to the process of customizing target region specific probes for sequences of one gene or more genes, hybridizing the probes with genomic DNA and enriching target gene DNA fragments. Specifically, it includes genomic DNA fragmentation, terminal repair and A base addition, then connecting adapter sequences and carrying out the first round of PCR amplification to obtain the whole genome library pre-library (whole genome library), then hybridizing the pre-library with the target region specific probes, washing the hybridized products and carrying out the second round of PCR amplification to obtain the final capture library. Before hybridization, the pre-library needs to be denatured and melted to form a single strand. All molecules of single-strand library comprise adapter sequences, which are about 60-70 bases, and the specific structure of which is shown in Figure 1. Therefore, during hybridization, the adapter sequences of molecules of single-strand library bound to the probes may bind to other single-strand library molecules of non-target regions, resulting in a decrease in specificity of capture, as shown in Figure 2. Therefore, it is necessary to block the adapter sequence (for example, the blocker scheme of the present disclosure) to improve the hybridization specificity, as shown in Fig. 3.

At present, the blockers mainly use the means of complementary pairing with the adapter sequences, and blocking of the adapter sequences makes no complementary pairing between the adapter sequences of library molecules, thus improving the specificity of capture. Current technologies mainly includes the following three types: the first type is a complete complementary pairing with an adapter sequence (comprising a sample label (index) sequence), and this type of blocker can effectively blocking complementing between the adapter sequences of library molecules, but the cost is high due to the variety of index sequences, and because the blockers have an index sequence, it will cause the blockers to act as primers for subsequent amplification when multiple samples are hybridized at the same time, resulting in confusion of index, sample crosstalk, false positive and other results; the second type is a complete complementary pairing with the adapter sequence (the corresponding position of the index sequence is modified by C3 spacer, etc.), and this type of blocker is prone to melt during subsequent hybridization at 65°C due to be unable to achieve completely complementary pairing, and leads to poor blocking effect; the third type is a complete complementary pairing with the adapter sequence (the corresponding position of the index sequence adopts degenerate bases), this type of blocker cannot guarantee that the number of sequences is sufficient to corresponding to the index one-to-one, due to the randomness of degenerate bases, and completely complementary pairing cannot be achieved, and this type of blocker is also prone to melt during subsequent hybridization at 65°C, which leads to poor blocking effect.

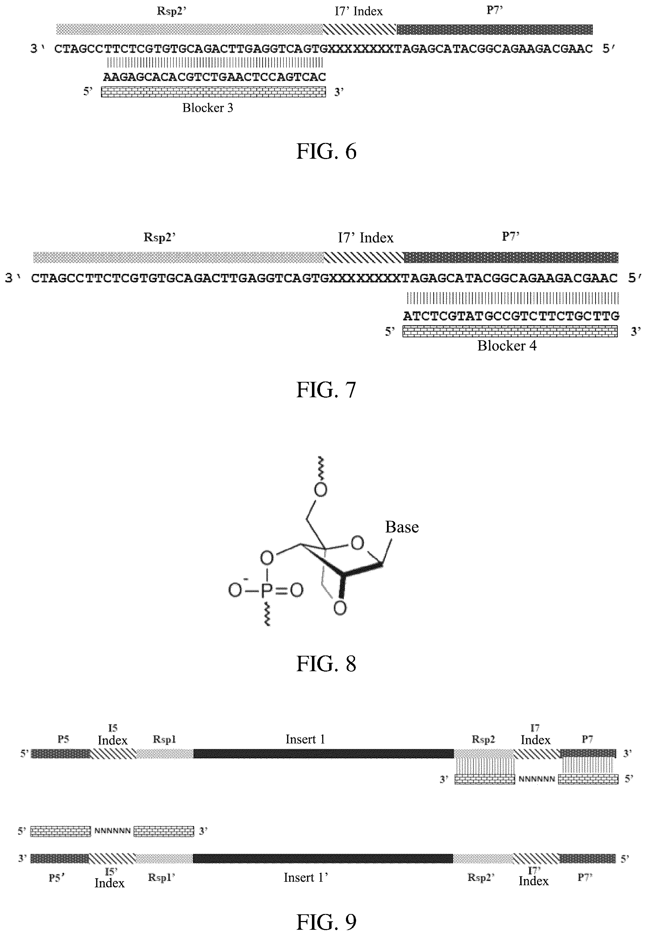

The non-complete adapter blocker sequences of the technical schemes of the present disclosure specifically comprises four segments of blockers, wherein, blocker 1 blocks P5 sequence at 5' end (shown in Fig. 4), blocker 2 blocks Rspl sequence (shown in Fig. 5), blocker 3 blocks Rsp2' sequence (shown in Fig. 6), blocker 4 blocks a portion of P7' sequence (shown in Fig. 7). Blockers 1 and 4 both comprise locked nucleic acid modification, and the structure of locked nucleic acid is shown in Fig. 8. Blockers 2 and 3 undergo reverse dT modification at 3' ends. The four blockers do not comprise an index sequence (effectively avoiding an index sequence), which ensures that there is no cross-contamination rate in subsequent multi-sample hybridization (Fig. 3); at the same time, each blocker is modified by locked nucleic acid to increase its annealing temperature, which ensured that the blocker can be effectively complementary pairing with an adapter sequence during subsequent hybridization at 65°C, to improve the hybridization specificity; blocker 3 is not completely complementary paired with Rsp2', and the starting position of the blocker is shown in Fig. 6, which further avoids the complementary pairing between the blockers and ensures the blocking effect of the blockers.

### Example 1

1. Sample preparation: the program of Bioruptor ultrasonic crusher was set to 30s on/30s off, 20 cycles. Samples were lung cancer ctDNA standard samples (5*%* EGFR L858R gDNA standard samples, GW-OGTS122) and NA12878 standard samples (negative control) produced by GeneWell. 4 aliquots of 100ng samples were added into 0.6 mL PCR tubes and numbered 1-8 (wherein 1-4 were EGFR standard samples with 5*%* mutation frequency, and 5-8 were NA12878), and then 4 aliquots of 10ng samples were added into 0.6 mL PCR tubes and numbered 9-16 (wherein 9-12 were EGFR standard samples with 5*%* mutation frequency, and 13-16 were NA12878), and TE buffer was supplemented to 50 µL, fully mixed and centrifuged. The samples above were divided into two batches and put into Bioruptor ultrasonic crusher to carry out DNA fragmentation. The average length of DNA fragments was controlled in the range of 200bp-250bp.
2. Preparation of pre-library (whole genome library): the kit for constructing library was VAHTS^{®} Universal DNA Library Prep Kit for Illumina V3 (ND607-02) from Vazyme, and the specific steps were as follows:
2.1. The End Prep Mix 4 was thawed, and then mixed by inversion, and the following reagent system was prepared in sterilized PCR tube:

**Table 1 End Prep Mix 4 System**

| Components | Volume (µL) |
|---|---|
| Input DNA(Fragment) | 50 |
| End Prep Mix4 | 15 |
| Total | 65 |

The mixture above was gently pipetted to mix, and centrifuged briefly, and placed into PCR instrument for the following reaction:

**Table 2 Program for PCR**

| Temperature | Time |
|---|---|
| Hot cover 100°C | On |
| 20°C | 15 min |
| 65°C | 15 min |
| 4°C | Keep |

2.2. immediately after the reaction was completed, the following reagent system was prepared:

**Table 3 Ligation system**

| Components | Volume (µL) |
|---|---|
| Product of previous step | 65 |
| Rapid Ligation buffer 2 | 25 |
| Rapid DNA ligase | 5 |
| DNA Adapter | 5 |
| Total | 100 |

The mixture above was gently pipetted to mix and centrifuged briefly, and placed into PCR instrument for the following reaction:

**Table 4 Program for PCR**

| Temperature | Time |
|---|---|
| Hot cover 100°C | On |
| 20°C | 15 min |
| 4°C | Keep |

2.3. Purification of the ligated product: 60 µL purification magnetic beads from Vazyme were added into the ligated product above, and the mixture above was fully mixed, stood at room temperature for 5min, placed on a magnetic rack for about 5min to completely adsorb the magnetic beads until the solution was clarified, and the supernatant was carefully removed; and 200 µL freshly prepared 80*%* ethanol was added to rinse the residue, the residue above was incubated at room temperature for 30s-60s, and the supernatant was carefully removed, and repeated once; after the magnetic beads were dried, they were eluted by adding 22 µL ultrapure water, placed at room temperature for 3min, and then placed on a magnetic rack. After the solution was clarified, 20 µL supernatant was pipetted for later use.
2.4. Amplification of Product: PCR Primer Mix 3 and VAHTS HiFi Amplification Mix were thawed, and then mixed by inversion, and the following reaction was prepared in sterilized PCR tube:

**Table 5 Amplification reaction system**

| Components | Volume (µL) |
|---|---|
| Product of previous step | 20 |
| VAHTS HiFi Amplification mix | 25 |
| PCR primer solution (i5&i7) | 5 |
| Total | 50 |

The mixture above was gently pipetted to mix, and centrifuged briefly, and placed into PCR instrument for the following reaction:

**Table 6 Program for PCR**

| Temperature | Time | note |
|---|---|---|
| Hot cover 100°C | On | |
| 95°C | 3 min | |
| 98°C | 20s | 7-10 Cycles |
| 60°C | 15s | |
| 72°C | 30s | |
| 72°C | 5min | |
| 4°C | Keep | |

2.5. 50 µL purification magnetic beads from Vazyme were added into the ligated product above, and the mixture above was fully mixed, stood at room temperature for 5min, placed on a magnetic rack for about 5min to completely adsorb the magnetic beads until the solution was clarified, and the supernatant was carefully removed; and 200 µL freshly prepared 80*%* ethanol was added to rinse the residue, the residue above was incubated at room temperature for 30s-60s, and the supernatant was carefully removed, and repeated once; after the magnetic beads were dried, they were eluted by adding 22 µL ultrapure water, placed at room temperature for 3min, and then placed on a magnetic rack. After the solution was clarified, 20 µL supernatant was pipetted for later use. The 16 samples were numbered as Pre-libraries 1-16, and the index were numbered as 1-16 respectively. The sequence indexes were shown in Table 7.

**Table 7 the index sequences**

| Numbers of the Sample | i5 index (Novaseq v1.0,Hiseq2000/2500, Miseq) | i5 index (Novaseq v1.5,Hiseq3000/4000, Miniseq) | i7 index (all Illumina systems) |
|---|---|---|---|
| 1 | CGACCATT | AATGGTCG | GCCTATCA |
| 2 | GATAGCGA | TCGCTATC | CTTGGATG |
| 3 | AATGGACG | CGTCCATT | AGTCTCAC |
| 4 | CGCTAGTA | TACTAGCG | CTCATCAG |
| 5 | TCTCTAGG | CCTAGAGA | TGTACCGT |
| 6 | ACATTGCG | CGCAATGT | AAGTCGAG |
| 7 | TGAGGTGT | ACACCTCA | CACGTTGT |
| 8 | AATGCCTC | GAGGCATT | TCACAGCA |
| 9 | CTGGAGTA | TACTCCAG | CTACTTGG |
| 10 | GTATGCTG | CAGCATAC | CCTCAGTT |
| 11 | TGGAGAGT | ACTCTCCA | TCCTACCT |
| 12 | CGATAGAG | CTCTATCG | ATGGCGAA |
| 13 | CTCATTGC | GCAATGAG | CTTACCTG |
| 14 | ACCAGCTT | AAGCTGGT | CTCGATAC |
| 15 | GAATCGTG | CACGATTC | TCCGTGAA |
| 16 | AGGCTTCT | AGAAGCCT | TAGAGCTC |

3. Preparation of Target Region Capture Library:
3.1. Preparation of the blockers. The blockers were synthesized by Sangon Biotech (Shanghai) Co., Ltd., and the specific sequences were shown in Table 2:

**Table 8 Sequences of the blockers**

| Names of the blockers | Sequences (5 '-3') | Modification |
|---|---|---|
| Blocker 1 (SEQ ID No.1) | TGTAGATCTCGGTGGTCGCCGTAT***C***ATT | Locked nucleic acid modification at position 25 base from the 5' end |
| Blocker 2 (SEQ ID No.2) | AGATCGGAAGAGCGTCGTGTAGGGAAA GAGTGT | reverse dT modification at the 3' end |
| Blocker 3 (SEQ ID No.3) | AAGAGCACACGTCTGAACTCCAGTCAC | reversed dT modification at the 3' end |
| Blocker 4 (SEQ ID No.4) | ATCTCGTATGCCGTCTTCT***G***C***T***TG | Locked nucleic acid modification at positions 20 and 22 bases from the 5' end |

Reverse dT was modified to an inverted deoxy T base, which binds to the 3' end of the oligonucleotide and inhibits the extension of DNA polymerase, that is, prevents the blockers from acting as primer to amplify products.

The blockers were prepared in powder form and dissolved in enzyme-free water at a concentration of 1000 µM/L.

3.2. Hybridization reaction: the hybridization-related reagents were purchased from Twist Bioscience, including customized probe Panel (interval size 420K), Fast Hybridization Mix, Hybridization Enhancer, Amplification Primers, Fast Binding Buffer, eluent 1/2 (Washing Buffer 1/2, Streptavidin Binding Beads and DNA Purification Beads. In addition, we purchased Cot1 DNA (15279101) and Kapa Hifi hotstart ready Mix (kk2601) ourselves. 187.5 ng samples from Pre-libraries 1, 2, 3, 4, 13, 14, 15, 16, respectively, were placed in a 1.5 ml centrifuge tube, fully mixed and centrifuged, labeled as hybrid library 1. Then the probe Panel, cot1 DNA and blocker 1, blocker 2, blocker 3, blocker 4 were thawed and mixed by inversion, and the following reagent system was prepared in sterilized PCR tube:

**Table 9 part component system in hybridization**

| Components | Volume (µL) |
|---|---|
| Hybridization Library 1 | 1500ng |
| Probe Panel | 4 |
| Blocker 1 | 2 |
| Blocker 2 | 2 |
| Blocker 3 | 2 |
| Blocker 4 | 2 |
| cot1 DNA | 2 |

The part component system above was fully mixed and centrifuged briefly, and then the sterilized PCR tube above was placed in a vacuum concentration centrifuge, the part component system in the sterilized PCR tube above was concentrated to dry powder, and then the following reagent system was prepared in the centrifuge tube:

**Table 10 part component system in hybridization**

| Components | Volume (µL) |
|---|---|
| Fast hybridization Mix | 20 |
| Hybridization Enhancer | 30 |
| Total | 50 |

The mixture above was gently pipetted to mix, centrifuge briefly, and placed into PCR instrument for the following reaction:

**Table 11 Program for PCR**

| Temperature | Time |
|---|---|
| 95°C | 5min |
| 60°C | 2h |

3.3. Cleaning streptavidin magnetic beads: 40 minutes before the completion of reaction of the previous step, Streptavidin magnetic beads at 4°C were taken out, equilibrated at room temperature for 30min.
1) 100 µL magnetic beads were pipetted and placed into a 1.5 ml low adsorption centrifuge tube and the magnetic beads were washed using a bead-binding buffer.
2) 200 µL bead-binding buffer was added into the centrifuge tube above, gently blown to mix for 10 times, centrifuged instantaneously, placed on a magnetic rack for several minutes, and the supernatant was discarded with a pipette when the solution was completely clarified. The centrifuge tube above was removed from the magnetic rack.
3) Step 2 was repeated twice.
4) 200µL magnetic bead suspension was added into that centrifuge tube, gently blown to mix, and all magnetic bead suspension was transferred to a new 1.5 mL Low adsorption PCR tube.

3.4. Elution of hybridization products: 200 µL magnetic bead suspension and hybridization products were fully mixed, incubated at room temperature for 30min, then washed with Eluent 1 at 65°C and incubated at 65°C for 5min, repeated 3 times; finally, washed with eluent 2, and incubated at 48°C for 5min and repeat for 3 times.

3.5. Amplification of elution product: Amplification Primers and Kapa Hifi Hotstart ReadyMix (kk2601) were thawed and mixed by inversion, and the following reagent system was prepared in sterilized PCR tube:

**Table 12 Elution System**

| Components | Volume (µL) |
|---|---|
| Elution product | 22.5 |
| KAPA HiFi Hotstart ReadyMix | 25 |
| Amplification primer | 2.5 |
| Total | 50 |

The mixture above was gently pipetted to mix, centrifuged briefly, and placed into PCR instrument for the following reaction:

**Table 13 Program for PCR**

| Temperature | Time | Cycle numbers |
|---|---|---|
| 98°C | 45s | 1 |
| 98°C | 15s | 14 cycles |
| 60°C | 30s | |
| 72°C | 30s | |
| 72°C | 1min | 1 |
| 4°C | Keep | |

3.6. purification of product: 90 µL purification beads were added into the product above, and the resulting mixture was fully mixed, stood at room temperature for 5min, placed on a magnetic rack for about 5min to completely adsorb the magnetic beads until the solution was clarified, and the supernatant was carefully removed; and 200 µL freshly prepared 80% ethanol was added to rinse the residue, and incubated at room temperature for 30s-60s, and the supernatant was carefully removed, and the rinse step was repeated once; after the magnetic beads were dried, they were eluted by adding 32 µL ultrapure water, placed at room temperature for 3min, and then placed on a magnetic rack. After the solution was clarified, 30 µL supernatant was pipetted for later use.

4. Sequencing on the instrument:
the library was diluted to 1ng/µL, and taken out 1 µL for detection on Agilent 4200 Tapestation system (Agilent Technologies of America); in addition, another 1 µL was taken out for qPCR detection, and the concentration of library loaded onto the instrument was determined according to the detection results. According to the concentration obtained in the previous step, the library was diluted to a concentration meeting the requirement for loading onto the instrument (2nmol), and PE150 sequencing was carried out on Novaseq sequencing platform of Illumina Company, with the data volume of each sample being 1.5 G.

In example 1, four blockers were used, and the blocking positions were all at the 5' ends of both strands of library DNA (Fig. 3). If the blockers of example 1 were subsequently used as primers, and there were no intermediate inserts in the amplified sequences, then there would not cause mutual contamination of samples.

### Example 2 (Comparative Example)

The construction method for pre-library of this example was the same as that of Example 1. The pre-libraries 1, 2, 3, 4, 5, 6, 7 and 8 were also selected in the subsequent preparation step of the target region capture library, with the only difference is that the blockers 1, 2, 3 and 4 were not added in the preparation step of the target region capture library, and the other experimental steps were the same.

### Example 3

The construction method for pre-library of this example was the same as that of Example 1. The pre-libraries 1, 2, 3, 4, 5, 6, 7 and 8 were also selected in the subsequent preparation step of the target region capture library, with the only difference is that the preparation steps of the target region capture library adopted blockers (including multiple blockers) which were completely complementary and paired with the adapter sequences of the pre-library, and the specific sequences of the blockers were shown in Table 14; and the other experimental steps were the same.

**Table 14 Blockers of prior art**

| Pre-library | Blockers (5'-3') |
|---|---|
| 1 | AATGATACGGCGACCACCGAGATCTACACAATGGTCGACACTCTTTCCC TACACGACGCTCTTCCGATCT；(SEQ ID No.5) GATCGGAAGAGCACACGTCTGAACTCCAGTCACTGATAGGCATCTCGTA TGCCGTCTTCTGCTTG(SEQ ID No.6) |
| 2 | AATGATACGGCGACCACCGAGATCTACACTCGCTATCACACTCTTTCCC TACACGACGCTCTTCCGATCT；(SEQ ID No.7) GATCGGAAGAGCACACGTCTGAACTCCAGTCACCATCCAAGATCTCGTA TGCCGTCTTCTGCTTG(SEQ ID No.8) |
| 3 | AATGATACGGCGACCACCGAGATCTACACCGTCCATTACACTCTTTCCC TACACGACGCTCTTCCGATCT；(SEQ ID No.9) GATCGGAAGAGCACACGTCTGAACTCCAGTCACGTGAGACTATCTCGTA TGCCGTCTTCTGCTTG(SEQ ID No.10) |
| 4 | AATGATACGGCGACCACCGAGATCTACACTACTAGCGACACTCTTTCCC TACACGACGCTCTTCCGATCT；(SEQ ID No.11) GATCGGAAGAGCACACGTCTGAACTCCAGTCACCTGATGAGATCTCGTA TGCCGTCTTCTGCTTG(SEQ ID No.12) |
| 5 | AATGATACGGCGACCACCGAGATCTACACCCTAGAGAACACTCTTTCCC TACACGACGCTCTTCCGATCT；(SEQ ID No.13) GATCGGAAGAGCACACGTCTGAACTCCAGTCACACGGTACAATCTCGTA TGCCGTCTTCTGCTTG(SEQ ID No.14) |
| 6 | AATGATACGGCGACCACCGAGATCTACAC*CGCAATGT*ACACTCTTTCCC TACACGACGCTCTTCCGATCT；(SEQ ID No.15) GATCGGAAGAGCACACGTCTGAACTCCAGTCAC*CTCGACTT*ATCTCGTA TGCCGTCTTCTGCTTG(SEQ ID No.16) |
| 7 | AATGATACGGCGACCACCGAGATCTACACACACCTCAACACTCTTTCCC TACACGACGCTCTTCCGATCT；(SEQ ID No.17) GATCGGAAGAGCACACGTCTGAACTCCAGTCACACAACGTGATCTCGTA TGCCGTCTTCTGCTTG(SEQ ID No.18) |
| 8 | AATGATACGGCGACCACCGAGATCTACACGAGGCATTACACTCTTTCCC TACACGACGCTCTTCCGATCT；(SEQ ID No.19) GATCGGAAGAGCACACGTCTGAACTCCAGTCACTGCTGTGAATCTCGTA TGCCGTCTTCTGCTTG(SEQ ID No.20) |
| 9 | AATGATACGGCGACCACCGAGATCTACACTACTCCAGACACTCTTTCCC TACACGACGCTCTTCCGATCT；(SEQ ID No.21) GATCGGAAGAGCACACGTCTGAACTCCAGTCACCCAAGTAGATCTCGTA TGCCGTCTTCTGCTTG(SEQ ID No.22) |
| 10 | AATGATACGGCGACCACCGAGATCTACACCAGCATACACACTCTTTCCC TACACGACGCTCTTCCGATCT；(SEQ ID No.23) GATCGGAAGAGCACACGTCTGAACTCCAGTCACAACTGAGGATCTCGTA TGCCGTCTTCTGCTTG(SEQ ID No.24) |
| 11 | AATGATACGGCGACCACCGAGATCTACACACTCTCCAACACTCTTTCCC TACACGACGCTCTTCCGATCT；(SEQ ID No.25) GATCGGAAGAGCACACGTCTGAACTCCAGTCACAGGTAGGAATCTCGT ATGCCGTCTTCTGCTTG(SEQ ID No.26) |
| 12 | AATGATACGGCGACCACCGAGATCTACACCTCTATCGACACTCTTTCCC TACACGACGCTCTTCCGATCT；(SEQ ID No.27) GATCGGAAGAGCACACGTCTGAACTCCAGTCACTTCGCCATATCTCGTA TGCCGTCTTCTGCTTG(SEQ ID No.28) |
| 13 | AATGATACGGCGACCACCGAGATCTACACGCAATGAGACACTCTTTCCC TACACGACGCTCTTCCGATCT；(SEQ ID No.29) GATCGGAAGAGCACACGTCTGAACTCCAGTCACCAGGTAAGATCTCGTA TGCCGTCTTCTGCTTG(SEQ ID No.30) |
| 14 | AATGATACGGCGACCACCGAGATCTACACAAGCTGGTACACTCTTTCCC TACACGACGCTCTTCCGATCT；(SEQ ID No.31) GATCGGAAGAGCACACGTCTGAACTCCAGTCACGTATCGAGATCTCGTA TGCCGTCTTCTGCTTG(SEQ ID No.32) |
| 15 | AATGATACGGCGACCACCGAGATCTACACCACGATTCACACTCTTTCCC TACACGACGCTCTTCCGATCT；(SEQ ID No.33) GATCGGAAGAGCACACGTCTGAACTCCAGTCACTTCACGGAATCTCGTA TGCCGTCTTCTGCTTG(SEQ ID No.34) |
| 16 | AATGATACGGCGACCACCGAGATCTACACAGAAGCCTACACTCTTTCCC TACACGACGCTCTTCCGATCT；(SEQ ID No.35) GATCGGAAGAGCACACGTCTGAACTCCAGTCACGAGCTCTAATCTCGTA TGCCGTCTTCTGCTTG(SEQ ID No.36) |

wherein, the sequences in italics and underlined were the index sequences.

### Example 4

The construction method for pre-library of this example was the same as that of Example 1. The pre-libraries 1, 2, 3, 4, 5, 6, 7 and 8 were also selected in the subsequent preparation step of the target region capture library, with the difference is that the preparation step of the target region capture library adopted blockers with N bases, as shown in Fig. 9, and the specific sequences of the blockers were shown in Table 15; and the other experimental steps were the same.

**Table 15 Blockers with N bases**

| Names of the blockers | Sequences (5'-3') |
|---|---|
| N-blockers | AATGATACGGCGACCACCGAGATCTACACNNNNNNNNACACTCTTTC CCTACACGACGCTCTTCCGATCT; (SEQ ID No.37) GATCGGAAGAGCACACGTCTGAACTCCAGTCACNNNNNNNNATCTCG TATGCCGTCTTCTGCTTG(SEQ ID No.38) |

wherein, bases in italics were degenerate base Ns, which were consisted of four bases: A/G/C/T.

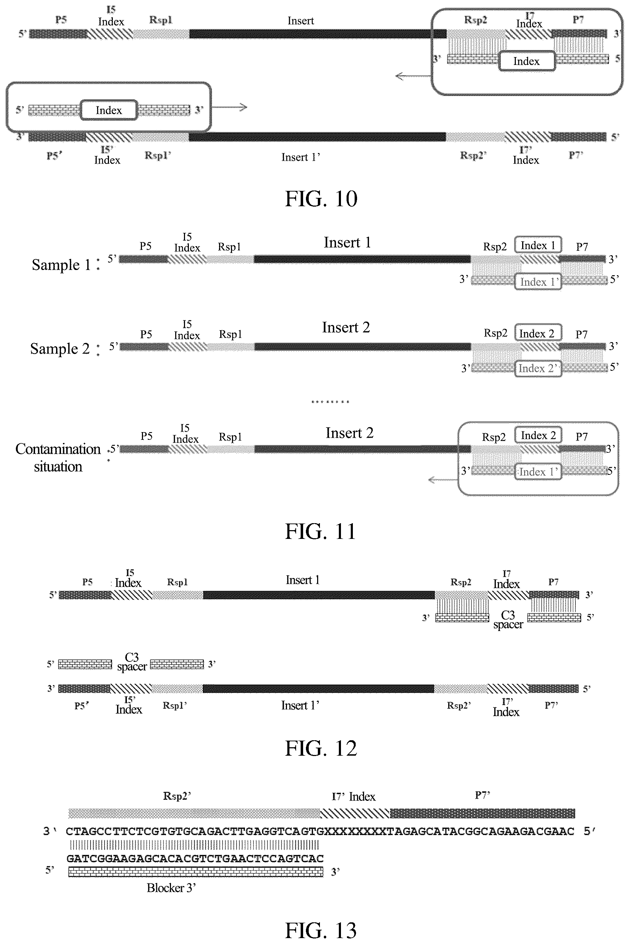

The blockers corresponding to the index one by one were used in Example 4. Blocking positions were both at the 3' ends of both strands of library DNA (Fig. 10). Hybridization reactions were carried out in multiple samples (libraries) at the same time, and each sample had a specific index sequence (ID), and accordingly there were the blockers containing the respective indexes. In the subsequent amplification and enrichment, if the blocker (containing the index 1') that should have otherwise blocking at sample 1 acted as the primer of sample 2, then after sequencing was completed, the data of the resolved index 1 (sample 1) was partly derived from sample 2, causing contamination (Fig. 12).

### Example 5

The construction method for pre-library of this example was the same as that of Example 1. The pre-libraries 1, 2, 3, 4, 5, 6, 7 and 8 were also selected in the subsequent preparation step of the target region capture library, with the difference is that the preparation step of the target region capture library adopted the blockers with C3 spacers (C3 spacers had 3 CH₂ groups, generally, one C3 was equivalent to one base in structure, and eight C3 spacers can be used to block eight bases of the index sequence, and upstream and downstream sequences that can block the sequences of P5/P7 and Rsp are connected to C3 spacers, which can serve as a connection to increase the spatial distance), as shown in Figure 12; and the other experimental steps were the same.

**Table 16 Blockers with C3 spacers**

| Names of the blockers | Sequences (5'-3') |
|---|---|
| C3 spacers-blockers | AATGATACGGCGACCACCGAGATCTACACC3spacerACACTCTTTCCCTA CACGACGCTCTTCCGATCT；(SEQ ID No.39) (SEQ ID No.40) GATCGGAAGAGCACACGTCTGAACTCCAGTCACC3spacerATCTCGTATG CCGTCTTCTGCTTG(SEQ ID No.41) (SEQ ID No.42) |

### Example 6

The construction method for pre-library of this example was the same as that of Example 1. The pre-libraries 1, 2, 3, 4, 5, 6, 7 and 8 were also selected in the subsequent preparation step of the target region capture library, with the difference is that in the preparation step of the target region capture library, Rsp2' sequence was completely covered by blocker 3, which was named as blocker 3', and blockers 1, 2 and 4 were unchanged, as shown in Fig. 13, the specific sequences of the blockers were shown in Table 6; and the other experimental steps were the same.

**Table 17 sequences of the blockers**

| Names of the blockers | Sequences (5'-3') | Modification |
|---|---|---|
| Blocker 1 | TGTAGATCTCGGTGGTCGCCGTAT***C***ATT | Locked nucleic acid modification at position 25 base from the 5' end |
| Blocker 2 | AGATCGGAAGAGCGTCGTGTAGGGAAAGAG TGT | reverse dT modification at the 3' end |
| Blocker 3' (SEQ ID No. 43) | GATCGGAAGAGCACACGTCTGAACTCCAGTC AC | reverse dT modification at the 3' end |
| Blocker 4 | ATCTCGTATGCCGTCTTCT***G***C***T***TG | Locked nucleic acid modification at positions 20 and 22 bases from the 5' end |

### Example 7

The construction method for pre-library of this example was different from that of Example 1, with the difference is that input amount of the samples was 10ng, so the pre-library 9, 10, 11, 12, 13, 14, 15 and 16 were selected in the subsequent preparation step of the target region capture library, and blockers 1, 2, 3 and 4 were selected in the preparation step of the target region capture library, and the other experimental steps were the same.

### The results of Experiments:

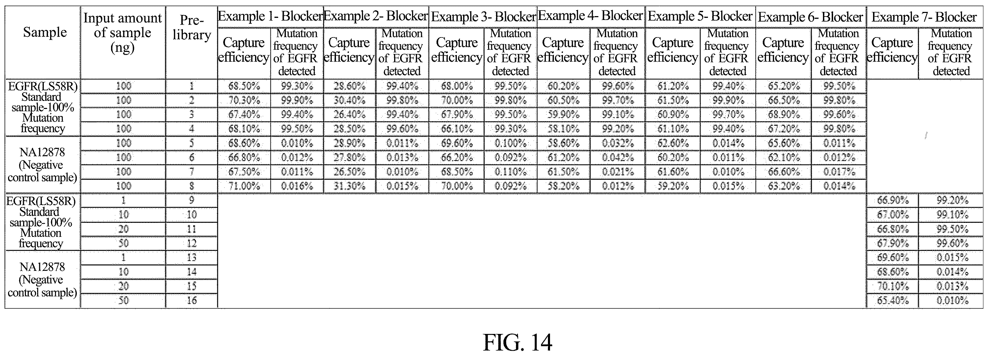

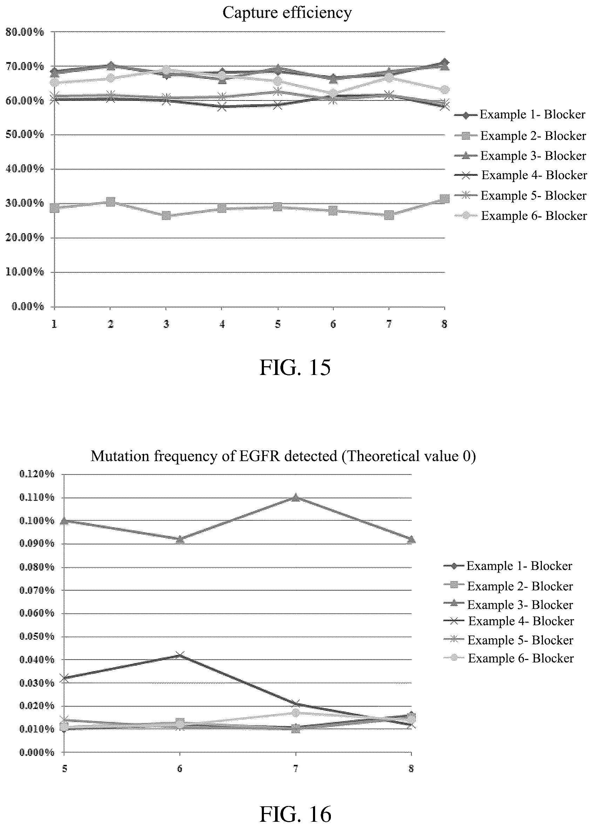

Results were referred to Figs. 14-16, blockers of Example 1 (blockers 1, 2, 3, 4) adopted in this disclosure had a capture efficiency of about 68*%*, and a crosstalk rate of about 0.01*%* for NA12878 negative reference sample, which was optimal compared with other Examples. Example 2 had a capture efficiency of about 28*%* because there were no blockers; the capture efficiency of Example 3 was close to that of Example 1, about 68*%*, but the crosstalk rate of sample was significantly higher (see the explanation below for details); the capture efficiency of Example 4 was about 60*%*, the reason was that in the blockers, N bases were used to block the index sequences, and the N bases were randomly consisted of A/G/C/T four bases, so only part of the blockers being completely complementary to the indexes can play a role, and the molar ratio of the effective blockers to the target sequence was reduced, resulting in the reduction of the blocking effect; the capture efficiency of Example 5 was about 60*%*, the C3 spacer modification was in the middle of the blockers, and its function was to cross the index sequence region. There was a state of non-base complementary pairing in the middle of the whole blocker. During the hybridization reaction at 65 °C, the blockers and the target sequences were unstable, which may lead to the decline of the blocking effect. Example 6 had a capture efficiency of about 66*%* and a crosstalk rate of sample of about 0.01*%*, and the effect was close to that of Example 1. Although bases 1-12 of the blocker comprising blocker 3' were the same as bases 2-13 of that blocker, there was no interaction and the final effect was not affected. The capture efficiency of Example 7 was about 67*%*, and the crosstalk ratio of sample was about 0.01*%*, which shows that different input amounts of sample do not affect the function of the blockers adopted in this scheme.

## Claims

1. A blocker, comprising blockers 1, 2, 3 and 4; wherein
the blocker 1 is complementary to a sequence of sequencing adapter P5 for blocking the sequence of sequencing adapter P5 at the 5' end of a first strand during the preparation of a target region capture library;
the blocker 2 is complementary to a sequence of Rspl for blocking the sequence of Rspl in 5' end to 3' end direction on the first strand during the preparation of the target region capture library;
the blocker 3 is partially complementary to a sequence of Rsp2' for blocking the sequence of Rsp2' in 5' end to 3' end direction on a second strand during the preparation of the target region capture library;
the blocker 4 is complementary to a sequence of sequencing adapter P7' for blocking the sequence of sequencing adapter P7' at the 5' end of the second strand during the preparation of the target region capture library.

2. The blocker according to claim 1, wherein the blocker 1 comprises a locked nucleic acid at position 25 base from the 5' end; the blockers 2 and 3 comprise a reverse dT modification at the 3' end; and the blocker 4 comprises locked nucleic acids at positions 20 and 22 bases from the 5' end.

3. The blocker according to any one of claims 1 to 2, wherein the blocker 3 is complementary to the Rsp2' sequence from the first base, at the 5' end, to the 27th base, at the 3' end direction.

4. The blocker according to any one of claims 1 to 2, wherein the blocker 1 is 28 bases in length, the blocker 2 is 33 bases in length, the blocker 3 is 27 bases in length, and the blocker 4 is 24 bases in length.

5. The blocker according to any one of claims 1 to 2, wherein the blocker 1 comprises the sequence set forth in SEQ ID No. 1, the blocker 2 comprises the sequence set forth in SEQ ID No. 2, the blocker 3 comprises the sequence set forth in SEQ ID No. 3 or SEQ ID No. 5, and the blocker 4 comprises the sequence set forth in SEQ ID No. 4.

6. A capture kit comprising the blocker of any one of claims 1 to 5.

7. A method of capturing a target region library, the method comprising capturing a target region from a library to be captured using the blocker according to any one of claims 1 to 5 or the capture kit according to claim 6.

8. The method according to claim 7, wherein the sample is fragmented prior to capturing the target region from the library to be captured; and, the fragments of the sample have an average length ranging from 200bp to 250bp.

9. The method according to claim 8, wherein the method further comprises subjecting the fragments of the sample to pre-library preparation.

10. The method according to claim 9, wherein the method further comprises adding an index sequence to the fragments of the sample.

11. A method for constructing a target region capture library, the method comprising the following steps:
fragmenting sequences of a sample;
preparing a pre-library;
preparing the target region capture library from the pre-library using the blocker according to any of claims 1 to 5 or the capture kit according to claim 6 to obtain the target region capture library;
performing PCR on the target region capture library to obtain a sequencing library.

12. The method according to claim 11, wherein, the method comprises:
fragmenting the sample into fragments of the sample with an average length ranging from 200bp-250bp;
adding an index sequence to the fragments of the sample;
blocking the fragments of the sample using the blockers or the capture kit;
performing PCR on the sequences blocked to obtain the sequencing library.

13. The method according to claim 11, wherein, when preparing a target region capture library, the molar ratio of the blockers to the sequences in the pre-library is (150: 1) to (160: 1).
